# FASCICULE DE BREVET EUROPEEN

(11) **EP 0 735 023 B1**
(45) Date de publication et mention de la délivrance du brevet: **02.08.2006**
(21) Numéro de dépôt: 96400624.1
(22) Date de dépôt: 22.03.1996
(51) Int. Cl.: C07C 313/04, C07C 303/02, C07C 309/80

(54) **Réactif et procédé pour la synthèse de dérivés organiques oxysulfurés et fluorés**
Reagenz und Verfahren zur Synthese von organischen oxysulfierten und fluorinierten Derivaten
Reagent and process for the synthesis of organic sulphoxylated and fluorinated derivatives

(30) Priorité: 24.03.1995 FR 9503515; 29.12.1995 FR 9515764
(43) Date de publication de la demande: 02.10.1996
(73) Titulaire: RHODIA CHIMIE, 92408 Courbevoie Cédex (FR)
(72) Inventeur: Forat, Gérard, 69003 Lyon (FR); Mas, Jean-Manuel, 69390 Millery (FR); Saint-Jalmes, Laurent, 69330 Meyzieu (FR)
(74) Mandataire: Bernasconi, Jean Raymond

(56) Documents cités:
- EP-A- 0 165 135
- FR-A- 2 593 808
- FR-A- 2 660 923
- JOURNAL OF ORGANIC CHEMISTRY, vol. 54, no. 10, 12 Mai 1989, WASHINGTON, DC, US, pages 2452-2453, XP002007102 M. TORDEUX, ET AL.: "Reactions of bromotrifluoromethane and related halides. 8. Condensations with dithionite and hydroxymethanesulphinate salts"
- JOURNAL OF FLUORINE CHEMISTRY, vol. 45, no. 3, Décembre 1989, LAUSANNE, CH, pages 431-433, XP002007099 G.P. STAHLY: "Trifluoromethylation of 1,3,5-trinitrobenzene"

## Description

La présente invention concerne un procédé de préparation d'acides fluoroalcane sulfiniques et sulfoniques et de leurs sels.

Elle concerne plus particulièrement la préparation d'acides polyhalogénosulfinique et sulfonique, notamment difluoro- ou trifluorométhane sulfinique et sulfonique.

Les acides perhalogénoalcane sulfoniques et plus particulièrement l'acide trifluorométhane sulfonique sont utilisés comme catalyseurs ou comme intermédiaires en synthèse organique.

Initialement, le seul procédé connu de fabrication de l'acide trifluorométhane sulfonique étaient la fluoration électrochimique telle que décrite notamment par R. D. Howels, J. D. Mc Cown dans Chemical Reviews, 1977, 77, 69.

On connaît également le procédé de préparation d'acide trifluorométhanesulfinique, décrit dans le brevet européen publié sous le numéro EP-165 135, qui consiste à mettre en présence de bioxyde de soufre un métal choisi parmi le zinc, l'aluminium, le manganèse, le cadmium, le magnésium, l'étain et le fer, voire le nickel et le cobalt, dans un solvant aprotique polaire puis à ajouter un halogénure de trifluorométhyle sous une pression supérieure à 10⁵ Pa. Ce procédé permet d'obtenir un produit sous forme de trifluorométhane sulfinate avec de bons rendements. Le sulfinate obtenu se trouve dans un milieu contenant une quantité importante de sel de zinc. La séparation du sulfinate et des autres sels de zinc pose au niveau industriel un problème à résoudre.

Par ailleurs, cette technique, ainsi que celle décrite dans la demande française publiée sous le numéro 2 593 808, nécessitaient l'utilisation de bromures de perfluoroalcoyles qui sont réputés particulièrement nocifs pour les couches atmosphériques, notamment en raison de leur fort effet de serre et de leur effet réputé néfaste sur l'ozone.

C'est pourquoi l'un des buts de la présente invention est de fournir un réactif pour la préparation de dérivés organiques oxysulfurés et fluorés, par réaction avec un oxyde de soufre, qui permette d'utiliser des produits moins nocifs pour l'environnement que le bromure de trifluorométhyle tout en restant de prix peu élevé.

On a souvent cherché à utiliser comme source de radicaux perfluoroalcoyle, plus généralement de radicaux trifluorométhyle, des acides perfluorocarboxyliques, en mettant en oeuvre des réactions de décomposition visant à éliminer le fragment carboxylique desdits acides en libérant du bioxyde de carbone. Toutefois, les succès qui avaient été obtenus étaient très mitigés et utilisaient des systèmes catalytiques particulièrement compliqués. Les radicaux perfluoroalcoyle ou leurs équivalents engendrés par la décomposition desdits acides perfluorocarboxyliques étaient en outre instables dans le milieu réactionnel et nécessitaient l'emploi d'agents stabilisants.

La présente invention se propose d'obvier aux inconvénients des procédés existants en fournissant un réactif plus respectueux de l'environnement et capable de conduire aux produits désirés avec un rendement satisfaisant.

Au cours de l'étude qui a mené à la présente invention, il a été démontré qu'il était possible de générer des radicaux fluoroalcoyle à partir d'un acide fluorocarboxylique, sans catalyseur et sans agént susceptible de stabiliser les divers intermédiaires envisagés obtenus lors de la décomposition des différents acides perfluorocarboxyliques.

Il est apparu que, pour obtenir ainsi une décarboxylation des acides fluorocarboxyliques, deux conditions étaient essentielles ; l'une est le choix du solvant, et l'autre la teneur en impuretés du mélange constituant le réactif selon la présente invention.

Ainsi, il a pu être démontré le rôle absolument critique de la teneur en atomes d'hydrogène labiles du système, ou plus exactement en protons libérables, qui doit être inférieure à la teneur en groupes fluorés libérés par la décomposition des acides fluorocarboxyliques.

Par atome d'hydrogène labile ou proton libérable, on entend un atome d'hydrogène qui est susceptible d'être arraché sous forme de proton par une base forte.

En pratique, il s'agit des protons des fonctions acides qui présentent un pKa inférieur à environ 20 (par "environ", on souligne que le nombre 20 ne présente qu'un chiffre significatif).

Les buts cités précédemment, et d'autres qui apparaîtront par la suite, sont donc atteints au moyen d'un réactif pour la synthèse de dérivés organiques oxysulfurés et fluorés par réaction avec un oxyde de soufre, notamment du bioxyde de soufre, caractérisé par le fait qu'il comprend :
a) un acide fluorocarboxylique de formule Ea-CF₂-COOH où Ea est un atome ou un groupe électroattracteur, au moins partiellement salifié par un cation organique ou minéral, et
b) un solvant aprotique ; et
par le fait que la teneur en protons libérables portés par ses divers composants, y compris leurs impuretés, est au plus égale à la moitié de la concentration molaire initiale dudit acide fluorocarboxylique.

Plus la teneur en protons libérables sera faible, moins il y aura de risque dé réaction parasite et meilleur sera le rendement.

Ainsi, il est préférable que, dans le réactif, la teneur en atomes d'hydrogène labiles soit au plus égale à 10%, de préférence à 1% (en moles), par rapport à la teneur initiale en ledit acide fluorocarboxylique.

La principale impureté, porteuse d'atomes d'hydrogène labiles, est en général l'eau qui est susceptible de libérer jusqu'à deux protons par molécule.

D'une manière générale il est préférable d'utiliser des réactifs et des solvants soigneusement déshydratés, de manière que la teneur pondérale en eau du réactif soit au plus égale à 1 pour 1000, par rapport à la masse totale du réactif. En fonction de l'ensemble des conditions réactionnelles, de telles teneurs en eau peuvent être satisfaisantes, mais dans certains cas, il peut être intéressant d'opérer à des niveaux plus bas, par exemple de l'ordre de 1 pour 10 000.

Toutefois, il n'est pas nécessairement indispensable d'éliminer la totalité de l'eau et un rapport molaire eau/acide fluorocarboxylique inférieur à 10 % peut être toléré.

Par ailleurs, il a pu être montré que d'autres éléments, à savoir les éléments de transition ayant deux états de valence stables, tels le cuivre, pouvaient n'être pas fastes, voire pouvaient être néfastes pour l'invention.

Bien que ce réactif selon l'invention ne nécessite pas de catalyseur, de tels éléments métalliques peuvent être présents en tant qu'impuretés apportées notamment par le solvant.

Ainsi, il est préférable que la teneur molaire en ces éléments soit inférieure à 1000, avantageusement à 100, de préférence à 10 ppm par rapport à la teneur initiale en ledit acide fluorocarboxylique.

Pour favoriser certains substrats et favoriser certains types de réaction, il avait également été de nombreuses fois préconisé d'utiliser avec l'acide perfluoroacétique des éléments de la colonne VIII de la classification périodique des éléments. Cela s'est révélé sans intérêt pour la réaction visée ci-dessus. C'est pourquoi, compte tenu du prix élevé de ces composés, il est préférable d'utiliser des réactifs ne contenant pas de métaux de la colonne VIII, notamment des métaux de la mine du platine qui est le groupe constitué du platine, de l'osmium, de l'iridium, du palladium, du rhodium et du ruthénium.

Dans la présente description, il est fait référence au supplément au bulletin de la Société Chimique de France numéro 1, janvier 1966, où une classification périodique des éléments a été publiée.

Ainsi il est préférable que la teneur en métaux de la mine du platine, voire en métaux de la colonne VIII, soit inférieure à 100 ppm, avantageusement à 10 ppm, de préférence à 1 ppm. Ces valeurs s'entendent par rapport à l'acide fluorocarboxylique de départ et sont exprimées en moles.

D'une manière plus générale et plus empirique, on peut indiquer que ces deux catégories de métaux, à savoir les éléments de transition à deux états de valence et les éléments de la colonne VIII, doivent être présents dans le réactif à un niveau de concentration globale au plus égal à 1000 ppm molaires, de préférence à 10 ppm molaires.

On notera que les différents métaux présents à un tel niveau de concentration globale sont en quantité extrêmement faible, et à cet égard, ils ne jouent aucun rôle catalytique. Leur présence n'améliore pas la cinétique de la réaction voire lui est néfaste lorsqu'ils sont présents en trop grande quantité.

L'utilisation, en plus des composants de réactifs précités, de fluorure alcalin ou de fluorure d'ammonium quaternaire, habituellement présents dans les systèmes réactifs utilisant les carboxylates fluorés, ne s'est pas révélée néfaste, mais elle s'est révélée de peu d'intérêt, en raison du fait qu'elle produit des effluents salins difficiles à traiter.

On note cependant que la présence de fluorures dans le milieu a tendance à limiter à la fois la transformation de l'acide fluorocarboxylique de départ et la décomposition du produit d'arrivée. Globalement, cet effet se montre plutôt positif, en faveur d'un meilleur rendement de transformation de l'acide fluorocarboxylique en le produit désiré, c'est-à-dire d'une bonne sélectivité de la réaction.

Cet effet a tendance à être d'autant plus important que le contre-cation du fluorure est volumineux. Des cations qui peuvent être envisagés sont les cations de métaux alcalin de rang supérieur à celui du sodium, en particulier potassium ou césium; ou bien les ions de type "onium", à savoir des cations formés par les éléments des colonnes VA et VIA (tels que définis dans le tableau de classification périodique des éléments publiés au supplément au Bulletin de la Société Chimique de France en janvier 1966), avec 4 ou 3 chaînes hydrocarbonées.

Parmi les oniums dérivant d'éléments de la colonne VA, les réactifs préférés sont les tétraalcoyl ou tétraaryl ammonium ou phosphonium. Le groupe hydrocarboné comporte avantageusement de 4 à 12 atomes de carbone, de préférence de 4 à 8 atomes de carbone. Les oniums dérivant de la colonne VIA sont de préférence dérivés d'éléments de numéro atomique supérieur à celui de l'oxygène.

Malgré les inconvénients qui ont été cités plus haut, la teneur en ions fluorure est un paramètre que l'on pourra considérer. Il peut toutefois être préférable de limiter cette teneur, en particulier la teneur initiale, afin de faciliter le traitement final du milieu réactionnel. Ainsi il est avantageux que la teneur en fluorure, que l'on qualifie de ionique, c'est-à-dire susceptible d'être ionisé dans le milieu polarisant du réactif, soit au plus égale à la concentration molaire initiale en ledit sel d'acide fluorocarboxylique, avantageusement à la moitié, de préférence au quart.

Ainsi qu'on l'a mentionné ci-dessus, le solvant joue un rôle important dans la présente invention et doit être aprotique, et avantageusement polaire et comporter très peu d'impuretés porteuses d'hydrogène acide.

Il est ainsi préférable que le solvant aprotique polaire utilisable ait un moment dipolaire significatif. Ainsi, sa constante diélectrique relative ε est avantageusement au moins égale à environ 5 (les zéros de position ne sont pas considérés comme des chiffres significatifs dans la présente description à moins qu'il en soit précisé autrement). De préférence l'ε est inférieur ou égal à 50 et supérieur ou égal à 5, et est notamment compris entre 30 et 40.

On préfère en outre que les solvants de l'invention soient susceptibles de bien solvater les cations, ce qui peut être codifié par l'indice donneur DN de ces solvants. Il est ainsi préférable que l'indice donneur DN de ces solvants soit compris entre 10 et 30. Ledit indice donneur correspond au ΔH (variation d'enthalpie), exprimé en kilo joule (kilo calorie) par mole, de l'association dudit solvant aprotique polaire avec le pentachlorure d'antimoine.

Selon la présente invention, il est préférable que le réactif ne présente pas d'hydrogène acide sur le ou les solvants polaires qu'il utilise. En particulier lorsque le caractère polaire du ou des solvants est obtenu par la présence de groupes électroattracteurs, il est souhaitable qu'il n'y ait pas d'hydrogène en alpha de la fonction électroattractrice.

De manière plus générale, il est préférable que le pKa correspondant à la première acidité du solvant soit au moins égal à environ 20 ("environ" soulignant que seul le premier chiffre est significatif), avantageusement au moins égal à environ 25, de préférence compris entre 25 et 35.

Le caractère acide peut également être exprimé par l'indice accepteur AN du solvant, tel qu'il est défini par Reichardt, "Solvents and solvent effects in Organic Chemistry", 2ème édition, VCH (RFA), 1990, pages 23-24. Avantageusement, cet indice accepteur AN est inférieur à 20, en particulier inférieur à 18.

Il est préférable que ledit acide ou sel d'acide fluorocarboxylique soit au moins partiellement, de préférence complètement, soluble dans le milieu constituant le réactif.

Les solvants donnant de bons résultats peuvent être notamment des solvants de type amide. Parmi les amides, on comprend aussi les amides à caractère particulier comme les urées tétrasubstituées et les lactames monosubstitués. Les amides sont de préférence substitués (disubstitués pour les amides ordinaires). On peut citer par exemple les dérivés de pyrrolidone, tels que la N-méthylpyrrolidone, ou encore le N,N-diméthyl-formamide, ou le N,N-diméthylacétamide.

Sont également avantageux des solvants tels que la 1,3-diméthyl-3,4,5,6 tétrahydro-2(1H)pyrimidinone (DMPU), la 1,3 diméthyl-2-imidazolidinone (DMI ou DMEU), ou le benzonitrile.

Une autre catégorie particulièrement intéressante de solvant est constituée par les éthers, qu'ils soient symétriques ou non symétriques, qu'ils soient ouverts ou non. Dans la catégorie des éthers doivent être incorporés les différents dérivés des éthers de glycol tels que les différents glymes, le diglyme par exemple.

Dans l'acide fluorocarboxylique du constituant a) du réactif de l'invention, l'entité Ea qui exerce un effet électroattracteur sur l'atome de carbone difluoré est de préférence choisie parmi les groupes fonctionnels dont la constante de Hammett σₚ est au moins égale à 0,1. Il est en outre préférable que la composante inductive de σₚ, σᵢ, soit au moins égale à 0,2, avantageusement à 0,3. A cet égard, on se référera à l'ouvrage de March, "Advanced Organic Chemistry", troisième édition, John Wiley and Son, pages 242 à 250, et notamment au tableau 4 de cette section.

Plus particulièrement, l'entité électroattractrice Ea peut être choisie parmi les atomes d'halogène, de préférence légers, notamment de chlore et de fluor. L'acide fluorocarboxylique correspondant est un acide halogénofluoroacétique de formule (1) X - CF₂ - COOH où X est un atome d'halogène, avantageusement léger (chlore ou fluor).

Ea peut également être avantageusement choisie parmi les groupements nitriles (avec le risque comme réaction parasite d'une alpha élimination), carbonylés, sulfonés et perfluoroalcoylés. Des acides fluorocarboxyliques de ce type qui peuvent être utilisés répondent à la formule (2) R - G - CF₂ - COOH où R - G représente un groupe nitrile ou bien G représente 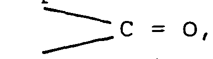 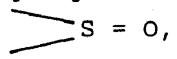 ou -(CF₂)ₙ- où n est supérieur ou égal à 1, et R représente un résidu organique ou minéral indifférent, de préférence un radical organique tel que aryle, alcoyle, ou aralcoyle, éventuellement substitué. R peut également représenter un support solide minéral ou organique, tel qu'une résine.

Dans le cas où G représente un groupe perfluoroalkylène -(CF₂)ₙ-, n est avantageusement compris entre 1 et 10, de préférence entre 1 et 5. Toujours dans ce cas, R peut également représenter un atome d'halogène, notamment le fluor.

De manière générale, sauf dans le cas où l'acide fluorocarboxylique est un polymère,le nombre total d'atomes de carbone de l'acide fluorocarboxylique n'excède avantageusement pas 50.

Les contre cations susceptibles de former un sel avec ledit acide fluorocarboxylique sont avantageusement volumineux. Ainsi, on préfère des sels alcalins, avantageusement où ledit métal est choisi parmi le sodium, le potassium, le rubidium, le césium et le francium. De préférence, le métal est d'une période dont le rang est au moins égal à celle du sodium, avantageusement à celle du potassium. On préfère également des sels d'ammonium quaternaire.

Il est également possible d'améliorer la réaction en utilisant des cations qui sont, soit naturellement volumineux comme les cations ammonium quaternaire ou phosphonium quaternaire, ou rendus volumineux par l'addition d'agents chélatants ou de préférence cryptands, tels que par exemple les éthers couronne ou les dérivés qui sont à la fois aminés et oxygénés.

Des sels d'acides perfluorocarboxyliques peuvent être avantageusement utilisés, tels que les trifluoroacétate, perfluoropropionate et perfluorobutyrate de métal alcalin, notamment de potassium.

On note que l'utilisation de séquestrants du type éthers couronnes accélère de façon marquée la transformation de l'acide fluorocarboxylique de départ.

De tels séquestrants pourront être utilisés avantageusement à raison de 5 à 100 % en mole, notamment de 5 à 25 % en mole par rapport à la teneur initiale en acide fluorocarboxylique.

Toutefois, certaines associations avec les autres partenaires du milieu réactionnel, notamment certains solvants, pourront avoir un effet moins favorable en ce qui concerne la stabilité du produit d'arrivée, et ne seront donc pas considérées comme avantageuses.

Un autre but de la présente invention est de fournir un procédé de synthèse de dérivés organiques oxysulfurés et fluorés, notamment de sels d'acides sulfiniques ou sulfoniques, utilisant le réactif selon la présente invention.

Ce but est atteint par :
a) la mise en présence dudit réactif avec un oxyde de soufre et
b) le chauffage du mélange résultant à une température comprise entre 100°C et 200°C, de préférence entre 120 et 150°C, et ce, pendant une durée d'au moins une demi-heure, avantageusement d'au moins une heure, et d'au plus une journée, avantageusement de moins de 20 heures.

La mise en présence, ou en contact du réactif avec le substrat peut être progressive ou non. En particulier on peut attendre que l'un des deux soit à la bonne température pour introduire l'autre. Cette introduction peut être progressive ou non. On peut couler le réactif dans le substrat ou réciproquement. On peut introduire le fluorocarboxylate et le substrat à la fois simultanément et progressivement dans le solvant.

Lorsque ledit oxyde est du bioxyde de soufre, le mélange résultant de l'étape a) peut comprendre deux phases en équilibre et comporter ainsi une phase liquide, où une partie au moins dudit acide et du bioxyde de soufre sont dissous dans ledit solvant, en équilibre avec une phase gazeuse qui contient du bioxyde de soufre.

En ce qui concerne les quantités relatives dudit acide fluorocarboxylique initial, et d'oxyde de soufre, de préférence bioxyde, il est préférable que le rapport soit compris entre 1 et 10, avantageusement aux alentours de deux, atomes de soufre par molécule d'acide fluorocarboxylique.

On a pu constater que, toutes choses étant égales par ailleurs, le rendement en le dérivé organique visé, dépend du degré d'avancement de la réaction et que l'on peut obtenir un rendement final très faible malgré une conversion importante des réactifs. Sans vouloir être lié à une quelconque théorie scientifique, il semble que tout se passe comme s'il y avait une cinétique de formation et une cinétique de dégradation des produits obtenus.

Pour éviter une dégradation trop importante du produit final, et donc assurer une bonne sélectivité de la réaction, il est préférable de ne pas chercher à convertir complètement l'acide fluorocarboxylique de départ. L'avancement de la réaction peut être contrôlé par le taux de transformation (TT) de l'acide qui est le rapport molaire de la quantité d'acide disparue sur la quantité d'acide initiale dans le milieu réactionnel, ce taux étant calculé aisément après dosage de l'acide restant dans le milieu.

De manière avantageuse, on ne conduira la réaction que jusqu'à l'obtention d'un taux de transformation de 40 à 80 %, de préférence de 50 à 70 %, puis on séparera les produits réactionnels. Il est possible d'atteindre ainsi une sélectivité de l'ordre de 80 % exprimée par le rapport molaire produit recherché/acide fluorocarboxylique transformé.

Pour se placer dans des conditions réactionnelles optimales, il est possible de limiter le taux de transformation en agissant à la fois sur la durée de la réaction, la nature du solvant et la présence d'additifs qui ont tendance à limiter cette transformation, tels que des ions fluorures par exemple. La cinétique de la réaction dépend, en outre, des partenaires réactionnels (acide fluorocarboxylique et oxyde de soufre) et le temps de réaction approprié pourra aisément être adapté au cas par cas en fonction de cette cinétique.

Dans le cas du bioxyde de soufre, une durée de réaction de 2 à 7 heures peut être suffisante, selon le réactif utilisé.

Une fois atteint le taux de transformation désiré, le mélange réactionnel peut être traité de façon connue en soi pour séparer le produit obtenu, les produits de départ pouvant être recyclés pour produire une quantité supplémentaire du dérivé organique visé.

Lorsque ledit oxyde de soufre est le bioxyde de soufre, le produit obtenu par chauffage du réactif est un acide sulfinique ou un sel d'acide sulfinique dont le contre-ion est celui du sel d'acide fluorocarboxylique de départ.

Pour séparer le produit de la réaction, une possibilité avantageuse consiste à procéder à une transformation supplémentaire en un dérivé relativement volatil et facilement distillable.

Ainsi, par exemple, au cours de la réaction entre SO₂ et l'acide trifluoroacétique CF₃CO₂H ou ses sels, l'acide trifluorométhylsulfinique CF₃SO₂H ou ses sels obtenus peuvent aisément être convertis en présence de chlore Cl₂ en le chlorure d'acide correspondant à une oxydation, à savoir CF₃SO₂Cl (cette réaction est générale aux acides utilisés et notamment aux acides perfluoro-alcanesulfiniques R_{f} SO₂H). Cette réaction qui n'affecte pas le réactif à base d'acide trifluoroacétique, permet avantageusement de séparer CF₃SO₂Cl par distillation en laissant des chlorures minéraux ainsi que le réactif de trifluorométhylation intact dans le milieu réactionnel, qui peut donc être réutilisé pour poursuivre la réaction avec l'oxyde de soufre. Cette réaction est commune aux différents acides sulfiniques fluorés que l'on peut obtenir selon l'invention. Cet exemple peut être généralisé à la séparation de tous types de dérivés organiques oxysulfurés fluorés obtenus selon l'invention susceptibles d'être transformés par une réaction appropriée en des produits plus volatils.

Pour passer de l'acide sulfinique à l'acide sulfonique correspondant, il convient de soumettre le produit réactionnel ou le produit de réaction purifié à une oxydation, en elle-même connue, notamment au moyen d'eau oxygénée ou d'hypochlorite de sodium. Un procédé de purification de trifluorométhylsulfinate de sodium, et d'oxydation en sulfonate, applicable selon l'invention est décrit dans la demande de brevet européen publiée sous le numéro EP-A-0 396 458.

Les sels d'acides sulfiniques ou sulfoniques ainsi obtenus peuvent être convertis en les acides libres correspondants en milieu acide.

Les produits de réaction, sels ou acides libres, peuvent être isolés aisément et mis en oeuvre dans des étapes ultérieures de synthèse organique. Ainsi, par exemple, on peut valoriser les chlorures de sulfinyle obtenus à partir d'acides sulfiniques fluorés préparés selon l'invention.

Les exemples non limitatifs suivants illustrent l'invention.

Les résultats présentés dans les exemples sont exprimés en fonction de trois grandeurs qui sont définies ci-après:
- le taux de transformation d'un réactif R (TTR) est le rapport de la quantité (molaire) de R disparue au cours d'une réaction sur la quantité de R initiale ;
- le rendement réel de production d'un produit P à partir d'un réactif R (RRP) est le rapport de la quantité de P produite sur la quantité de R initiale ;
- le rendement de transformation de R en P (RTP) qui est le rapport de la quantité de P produite sur la quantité de R disparue.

### EXEMPLE 1 : Préparation d'acide trifluorométhylsulfinique.

Dans un réacteur en Hastalloy de 100 ml, agité par une turbine, on introduit 42 g de N-méthylpyrrolidone (NMP), puis 5,32 g (35 mmol) de trifluoroacétate de potassium et enfin 4,9 g (76 mmol) de bioxyde de soufre gazeux par barbotage dans le liquide. Le bioxyde de soufre est totalement solubilisé par la NMP.

Le rapport molaire du bioxyde de soufre au trifluoroacétate de potassium est de 2,1.

La teneur en eau du mélange réactif est de 0,1 % en poids par rapport au poids du mélange, soit un rapport molaire de l'eau au trifluoroacétate de 0,07.

Le mélange est chauffé dans le réacteur fermé à une température de 140°C pendant 6 heures sous agitation.

Au cours de la réaction, la pression à l'intérieur du réacteur ramené à une température ambiante s'élève de 3,5.10⁵ Pa par rapport à la pression initiale.

Le milieu réactionnel est ensuite repris à l'eau et analysé par chromatographie ionique HPIC (High Performance Ionic Chromatography) en mode de séparation pour doser la transformation du trifluoroacétate de potassium.

Le taux de transformation (TT) du trifluoroacétate de potassium de départ, exprimé par le rapport molaire de la quantité de trifluoroacétate consommée (transformée) à la quantité initiale, est de 61,7 %.

Le rendement réel (RR), exprimé par le rapport molaire de la quantité de trifluorométhylsulfinate formée, sous forme libre ou salifiée, à la quantité de trifluoroacétate initiale, est de 29,7 %.

Le rendement par rapport au produit transformé (RT), exprimé par le rapport molaire de la quantité de trifluorométhylsulfinate formée, sous forme libre ou salifiée, à la quantité de trifluoroacétate transformée, est de 48,1 %. On isole le produit sous forme de sel de potassium.

### EXEMPLE 2

L'exemple 1 est exactement répété à ceci près qu'on utilise 8,6 g (35mmol) de trifluoroacétate de césium dans le réactif.

Le dosage par HPIC permet de calculer que TT vaut 68,4 %, RR vaut 21 % et RT vaut 30,7 %. On isole le produit sous forme de sel de césium.

Le trifluoroacétate de césium est d'emploi relativement moins avantageux que le sel de potassium.

### EXEMPLES 3 et 4

L'exemple 1 est exactement répété à ceci près qu'on utilise comme solvant le N,N-diméthylacétamide (DMAC, ε = 37,8) et le N,N-diméthylformamide (DMF, ε= 36,7), respectivement.

L'avancement de la réaction est dosé par HPIC et les résultats sont reportés dans le tableau 1, où l'on rappelle, pour chaque exemple, le solvant utilisé et son indice donneur D.

### Exemple comparatif 1

L'exemple 1 est exactement répété à ceci près que l'on opère seulement en présence d'un excès de bioxyde de soufre sans solvant (constante diélectrique ε = 14).

Les résultats figurent dans le tableau 1.

**TABLEAU 1**

| Exemple | Solvant | DN | TT | RR | RT |
|---|---|---|---|---|---|
| Ex 1 | NMP | 27,3 | 61,7 | 29,7 | 48,1 |
| Ex 3 | DMAC | 27,8 | 78,6 | 40,6 | 51,7 |
| Ex 4 | DMF | 26,6 | 80,4 | 33,8 | 41,7 |
| Ex comp 1 | -(SO₂)* | - | 9 | 0 | 0 |

| | | | | | |
|---|---|---|---|---|---|
| * SO₂ sert à la fois de solvant et de réactif. | | | | | |

L'Exemple comparatif 1 montre que le solvant est nécessaire à la transformation en le produit désiré.

### EXEMPLE 5

Cet exemple récapitule une série d'autres essais où différents solvants ont été testés dans des conditions proches de celles de l'exemple 1.

Le trifluoroacétate de potassium (dans un rapport pondéral CF₃CO₂K/solvant = 0,13) est mis en présence d'environ 2 équivalents molaires de bioxyde de soufre (rapport molaire SO₂/CF₃CO₂K de 1,9 à 2, 1).

Le mélange des réactifs est chauffé dans le réacteur fermé agité à 1000t/min, avec une montée en température de 10°C/min jusqu'à 140°C, pendant 6 h.

L'avancement de la réaction est dosé par HPIC et les résultats sont reportés dans le tableau 2 où l'on rappelle pour chaque essai le solvant utilisé, sa constante diélectrique ε, son indice donneur DN, son indice accepteur AN et la teneur en eau dans le milieu.

**TABLEAU 2**

| Essai | Solvant | ε | DN | AN | H₂O/CF₃ CO₂K % mol | TT (%) | RR CF₃ SO₂K (%) | RR F⁻ (%) | RT CF₃SO₂K (%) |
|---|---|---|---|---|---|---|---|---|---|
| a | DMSO | 48,9 | 29,8 | 19,3 | 4 | 26,6 | 4,6 | - | 17,3 |
| b | CH₃CN | 38 | 14,1 | 18,3 | 1,6 | 12,6 | 3 | 3 | 23,8 |
| c | DMF | 36,7 | 26,6 | 16,0 | 1 | 73,5 | 41,8 | 10,9 | 56,9 |
| d | NMP | 32,2 | 27,3 | 13,3 | 1,6 | 67,2 | 27, 9 | 25,1 | 41,5 |
| e | Benzonitrile | 25,2 | 11,9 | 15,5 | 1,5 | 22,1 | 7,4 | 3,6 | 33,5 |
| f | DMPU | 36,1 | / | / | 1,5 | 82,1 | 42,9 | 9,8 | 52,3 |
| g | DMAC | 37,8 | 27,8 | 13,6 | 1,9 | 74 | 43,4 | 3 | 58,6 |
| h | Anisole | 4,3 | / | / | 1,2 | 21,6 | 4,4 | 3 | 20,4 |
| i | Xylène | 2,4 | / | / | 0,4 | 23,2 | 4,4 | 3 | 19 |
| j | Diglyme | 5,7 | / | 9,9 | 1,9 | 28,4 | 15,6 | 3 | 54,9 |
| k | DMI (DMEU) | 37,6 | / | / | 1,4 | 66,1 | 47,5 | 7,4 | 72 |

De manière générale, pour les solvants peu acides (AN < 19), les rendements évoluent dans le même sens que la constante diélectrique ε. A cet égard, le DMF, le DMAC et le DMPU donnent d'excellents résultats, ceux de la NMP étant légèrement inférieurs.

Par contre, avec le DMSO et CH₃CN, les résultats sont moins bons, malgré des constantes diélectriques élevées, et cela est à rapprocher de leur caractère acide (AN = 19,3).

### EXEMPLE 6

L'exemple 1 est exactement répété à ceci près qu'on utilise des composés réactifs plus soigneusement déshydratés. La teneur en eau dans le mélange réactif est de 0,05 % en poids par rapport au poids du mélange, soit un rapport molaire de l'eau au trifluoroacétate de 0,04. Les résultats de l'essai, dosés par HPIC, sont reportés dans le tableau 3. Les résultats de l'exemple 1 sont également rappelés dans ce tableau.

### Exemple comparatif 2 (à rapprocher des exemples 1 et 6)

Inversement à l'exemple précédent, on répète l'exemple 1 avec des réactifs plus hydratés, de sorte que la teneur en protons libérables est hors des limites de l'invention. La teneur en eau dans le mélange réactif est de 0,8 % en poids par rapport au poids du mélange. Le rapport molaire de l'eau au trifluoroacétate est de 0,6, le rapport de la teneur en protons libérables apportés par l'eau à la teneur en trifluoroacétate est donc de 1,2. Les résultats de l'essai, dosés par HPIC, sont reportés dans le tableau 3.

### EXEMPLE 7

L'exemple 6 est exactement répété à ceci près qu'on utilise comme solvant le DMAC.

Les résultats de l'essai figurent dans le tableau 3, où sont également reportés les résultats de l'exemple 3.

**TABLEAU 3**

| Exemple | H₂O (% en poids) | H₂O/CF₃COOK (mol/mol) | TT (%) | RR (%) | RT (%) |
|---|---|---|---|---|---|
| Ex 1 | 0,1 | 0,07 | 61,7 | 29,7 | 48,1 |
| Ex 6 | 0,05 | 0,04 | 64 | 54 | 85 |
| Ex Comp 2 | 0,8 | 0,6 | 100 | 0 | 0 |
| Ex 3 | 0,1 | 0,07 | 78,6 | 40,6 | 51,7 |
| Ex 7 | 0,05 | 0,04 | 68 | 47 | 69 |

Les exemples 6 et 7 montrent qu'une faible teneur en eau améliore de façon remarquable le rendement de transformation.

L'exemple comparatif 2 confirme qu'une teneur en protons libérables du système réactif supérieure à la moitié de la teneur en sel d'acide trifluoroacétique est néfaste à la réaction de formation du trifluorométhylsulfinate.

### EXEMPLE 8

Cet exemple récapitule une série d'essais mettant également en évidence l'importance de la teneur en eau dans la réaction du bioxyde de soufre avec le trifluoroacétate de potassium dans des conditions proches de celles de l'exemple 1.

Toujours dans la NMP, le trifluoracétate de potassium (dans un rapport pondéral par rapport au solvant CF₃CO₂K/NMP = 0,13) est mis en présence d'environ 2 équivalents molaires de bioxyde de soufre (rapport molaire SO₂/CF₃CO₂K de 1,9 à 2,1).

Le mélange est chauffé dans le réacteur fermé agité à 1 000 t/min avec une montée en température de 10°C/min jusqu'à 140°C pendant 6 heures.

Les résultats sont résumés dans le tableau 4 suivant :

**TABLEAU 4**

| Essai | H₂O /CF₃COOK (mol %) | TT CF₃CO₂K (%) | RR CF₃SO₂K (%) | RT CF₃SO₂K (%) |
|---|---|---|---|---|
| a | 1,9 | 67,2 | 27,9 | 41,5 |
| b | 2,3 | 64,2 | 43,2 | 67,3 |
| c | 3,9 | 65,3 | 44,2 | 67,8 |
| d | 6,9 | 69,4 | 39,6 | 57,1 |
| e | 8,9 | 69,1 | 39,9 | 57,7 |

Dans ces essais, on observe la formation d'ions fluorure avec un rendement RR d'environ 25 %.

On constate une nette amélioration du rendement et de la sélectivité en passant des conditions a) aux conditions b). Un optimum apparait dans l'intervalle de 2 à 8 %, autour de 4 %.

### EXEMPLE 9

Cet exemple récapitule une série d'essais où des ions fluorure ont été introduits dans le milieu réactionnel dès le début de la réaction.

L'essai 9.a a été réalisé dans la NMP en suivant le protocole de l'exemple 5, essai d, et en ajoutant 1 mole de fluorure de potassium par mole d'acide trifluorocarboxylique de départ.

Les essais 9.b-d ont été réalisés dans le DMF en suivant le protocole de l'essai 5.c et en ajoutant différentes quantités de KF.

Les essais 9.e,f,g ont été réalisés dans les mêmes solvants en utilisant cette fois du fluorure de césium.

Les résultats sont rassemblés dans le tableau 5 suivant :

**TABLEAU 5**

| Essai | Solvant | Fluorure F⁻/CF₃CO₂K % mol | H₂O/CF₃CO₂K % mol | TT CF₃CO₂K % | RR CF₃SO₂K % | RT CF₃SO₂K % |
|---|---|---|---|---|---|---|
| 5.d | NMP | 0 | 1,7 | 67,1 | 27,9 | 41,5 |
| 9.a | " | KF 100 | 0,9 | 54,1 | 39,1 | 72,3 |
| 5.c | DMF | 0 | 1 | 73,5 | 41,8 | 56,9 |
| 9.b | " | KF 100 | 2 | 63,4 | 44,3 | 70 |
| 9.c | " | KF 10 | 1,4 | 73,8 | 45,9 | 62,2 |
| 9.d | " | KF 1 | 1,7 | 76 | 44,8 | 58,9 |
| 9.e | NMP | CsF 100 | | 57,7 | 37,4 | 64,8 |
| 9.f | " | CSF 10 | 1,3 | 67,2 | 42,9 | 63,8 |
| 9.g | DMF | CsF 100 | 1,9 | 62,6 | 46,2 | 73,8 |

Dans tous les cas, le taux de transformation de CF₃CO₂K est limité par la présence des fluorures et l'on observe une augmentation de la sélectivité et, en général, des rendements.

### EXEMPLE 10

Dans cet exemple, on compare les résultats obtenus en l'absence et en présence d'un éther couronne séquestrant, le 18 crown 6.

Différents essais ont été réalisés dans des solvants variés, suivant le protocole de l'exemple 6.

Les résultats sont résumés dans le tableau suivant.

**TABLEAU 6**

| Essai | Solvant | H₂O/ CF₃CO₂K (% mol) | 18 Cr6/CF₃CO₂K (% mol) | TT (%) | RR CF₃SO₂K (%) | RR F⁻ (%) | RT CF₃SO₂K (%) |
|---|---|---|---|---|---|---|---|
| 5.d | NMP | 1,9 | 0 | 67,2 | 27,9 | 25,1 | 41,5 |
| 10.a | NMP | 1,5 | 25 | 73,8 | 23,8 | 0 | 32,2 |
| | | | | | | | |
| 5.b | CH₃CN | 1,6 | 0 | 12,6 | 3 | 3 | 23,8 |
| 10.b | CH₃CN | 1,5 | 25 | 47,6 | 6,1 | 3 | 12,8 |
| | | | | | | | |
| 5.e | benzonitrile | 1,5 | 0 | 22,1 | 7,4 | 3,6 | 33,5 |
| 10.c | benzonitrile | 1,2 | 25 | 35,8 | 14,8 | 5,7 | 41,3 |
| | | | | | | | |
| 5.i | xylène | 0,5 | 0 | 23,2 | 4,4 | 3 | 19 |
| 10.d | xylène | 0,5 | 25 | 28,4 | 6,3 | 3 | 22,2 |

Dans tous les cas, la transformation du produit de départ est favorisée, sans incidence notable toutefois sur la décomposition en fluorures. Celle-ci est même diminuée avec le solvant NMP.

Dans les essais b,c et d, le rendement réel en CF₃SO₂K est bien meilleur quand on utilise le séquestrant.

### EXEMPLE 11

Cet exemple présente une étude cinétique de la réaction de l'essai 5.d.

Le taux de transformation du trifluoracétate, le rendement réel et le rendement de transformation du trifluorométhylsulfinate ainsi que le rendement réel en ions fluorure ont été déterminés pour des temps de réaction variant entre 2 et 9 h30.

Les résultats sont résumés dans le tableau 7. ci-après.

**TABLEAU 7**

| Essai | Temps (h) | H₂O/CF₃ CO₂K % mol | TT % | RR CF₃SO₂K % | RR F⁻ % | RT CF₃SO₂K % |
|---|---|---|---|---|---|---|
| 11.a | 2 | 1,3 | 46,2 | 23,5 | 3 | 51 |
| 11.b | 4 | 1,2 | 52,7 | 42,1 | 3 | 79,9 |
| 5.d | 6 | 1,9 | 67,2 | 27,9 | 25,1 | 41,5 |
| 11.c | 9h30 min | 1,3 | 81,4 | 4,1 | 83,3 | 5 |

On observe un maximum de rendement et de sélectivité vers 4 h de réaction.

Lorsque la durée de réaction augmente, le rendement chute et l'on voit apparaître une quantité d'ions fluorure croissante, signe de dégradation des groupements trifluorométhyle dans le milieu.

### Exemple 12 : Préparation d'acide pentafluoroéthylsulfinique.

Dans le même réacteur que celui de l'exemple 1, on introduit 40 g de NMP, 7,07 g de C₂F₅COOK anhydre (35 mmol) puis 4,9 g (76 mmol) de SO₂.

Le mélange est chauffé dans le réacteur fermé à une température de 140°C pendant 6 heures.

La variation de pression à l'intérieur du réacteur entre le début et la fin de la réaction est de 3,5 bar.

Le milieu réactionnel est repris à l'eau puis le mélange est dosé par RMN ¹⁹F.

Le taux de transformation TT vaut 85 %, le rendement de réaction RR vaut 73 % et le rendement de transformation RT vaut 86,2 %. On isole le produit sous forme de sel de potassium.

### EXEMPLE 13 : Préparation d'acide hepta fluoropropylsulfinique

Dans le même réacteur que celui de l'exemple 1, on introduit 40 g de NMP, 8,8 g de C₃F₇COOK anhydre (35 mmol) puis 4,9 g (76 mmol) de SO₂.

Le mélange est chauffé dans le réacteur fermé à une température de 140°C pendant 1 h 30.

La variation de pression à l'intérieur du réacteur entre le début et la fin de la réaction est de 45 Pa (4,5 bar).

Le milieu réactionnel est repris à l'eau puis le mélange est dosé par RMN ¹⁹F.

Le taux de transformation TT vaut 85 %, le rendement de réaction RR vaut 70 % et le rendement de transformation RT vaut 82 %.

On isole le produit sous forme de sel de potassium.

### EXEMPLE 14 : Préparation de chlorure de trifluorométhylsulfinyle

On prépare du trifluorométhylsulfinate de potassium dans les conditions de l'exemple 4.

Le DMF est éliminé du mélange réactionnel par distillation sous vide à une température ne dépassant pas 55-60°C.

Le résidu de distillation est repris à l'acétonitrile puis filtré. Le filtrat est distillé pour éliminer le solvant et on isole le trifluorométhylsulfinate de potassium avec un rendement de purification de 96 % par rapport au mélange réactionnel brut, dosé par chromatographie ionique.

Le produit résultant de cette opération est repris au toluène et additionné de chlorure de thionyle so Cl₂ en quantité stoechiométrique par rapport au trifluorométhylsulfinate. Le chlorure de trifluorométhylsulfinyle (CF₃SOCl) est obtenu avec un rendement de 65 %.

### EXEMPLE 15 : préparation de chlorure de trifluorométhylsulfonyle.

On prépare du trifluorométhanesulfinate de potassium dans les conditions de l'exemple 4.

Le DMF est éliminé du mélange réactionnel par distillation sous vide à une température ne dépassant pas 60°C.

Le résidu de distillation est repris avec de l'eau.

On fait barboter dans la solution aqueuse le chlore en quantité stéchiométrique par rapport au trifluorométhylsulfinate présent dans le milieu.

La température de réaction est de 0° - 5°C.

Par décantation de la couche inférieure on isole le chlorure de trifluorométhylsulfonyle.

Ce brut est distillé, eb : 28-31°C. Le rendement est de 80 % par rapport au trifluorométhylsulfinate présent dans le milieu.

### EXEMPLE 16 : Préparation de l'acide trifluorométhylsulfonigue (acide triflique).

La solution aqueuse obtenu dans les mêmes conditions que celle décrites dans l'exemple 15 est oxydée par de l'eau oxygénée 30 volumes. Un excès de 10 % d'eau oxygénée par rapport au trifluorométhylsulfinate de potassium est nécessaire.

La température de réaction est de 5°C.

Après distillation de l'eau et séchage. On acidifie les sels obtenus par de l'acide sulfurique 100 %. On sépare ainsi l'acide triflique de l'acide trifluoroacétique.

## Revendications

1. Procédé de synthèse d'un sel d'un composé organique à la fois fluoré et oxysulfuré, **caractérisé en ce qu'**il comporte les étapes :
a) de mise en présence d'un réactif comprenant :
a) un acide fluorocarboxylique de formule
Ea - CF₂ - COOH, où Ea représente un atome ou un groupe électroattracteur, au moins partiellement salifié par un cation organique ou minéral, et
b) un solvant aprotique ;
et **en ce que** la teneur en protons libérables portés par ses divers composants, y compris leurs impuretés, est au plus égale à la moitié de la concentration molaire initiale dudit acide fluorocarboxylique,
avec un oxyde de soufre ; et
b) de chauffage du mélange résultant à une température comprise entre 100 et 200°C, pendant une durée comprise entre 1/2 heure et 20 heures.

2. Procédé selon la revendication 1, **caractérisé en ce que** ladite teneur en protons du réactif est au plus égale à 10% de la concentration molaire en ledit acide fluorocarboxylique.

3. Procédé selon la revendication 1, **caractérisé en ce que** la teneur en eau dudit réactif est inférieure à 10 % de la concentration molaire en ledit acide fluorocarboxylique.

4. Procédé selon l'une quelconque des revendications 1 à 3, **caractérisé en ce que** la teneur en éléments de transition présentant au moins deux états de valence stables dudit réactif est inférieure à 1000 ppm molaires, avantageusement 100 ppm molaires, de préférence 10 ppm molaires par rapport audit acide fluorocarboxylique.

5. Procédé selon l'une quelconque des revendications précédentes, **caractérisé en ce que** la teneur en éléments de la colonne VIII de la classification périodique des éléments dudit réactif est inférieure à 100 ppm molaires, par rapport audit acide fluorocarboxylique.

6. Procédé selon l'une quelconque des revendications précédentes, **caractérisé en ce que** la teneur, exprimée en équivalent, en fluorure ionique dudit réactif est au plus égale à la concentration molaire dudit acide fluorocarboxylique.

7. Procédé selon la revendication 6, **caractérisé en ce que** le contre-cation du fluorure dudit réactif est choisi parmi les cations de métaux alcalins de rang supérieur à celui du sodium, en particulier potassium ou césium, ou bien les ions de type "onium".

8. Procédé selon l'une quelconque des revendications précédentes, **caractérisé en ce que** l'indice donneur du solvant aprotique dudit réactif est compris entre 10 et 30.

9. Procédé selon l'une quelconque des revendications précédentes, **caractérisé en ce que** l'indice accepteur du solvant aprotique dudit réactif est inférieur à 20.

10. Procédé selon l'une quelconque des revendications précédentes, **caractérisé en ce que** le pKa correspondant à la première acidité du solvant aprotique dudit réactif est au moins égal à 20.

11. Procédé selon l'une quelconque des revendications précédentes, **caractérisé en ce que** ledit réactif comprend un éther couronne séquestrant.

12. Procédé selon l'une quelconque des revendications précédentes, **caractérisé en ce que** l'atome ou .groupement électroattracteur dudit réactif est choisi parmi les groupes électroattracteurs dont la constante de Hammett σₚ est au moins égale à 0,1.

13. Procédé selon l'une quelconque des revendications précédentes, **caractérisé en ce que** ledit acide dudit réactif est choisi parmi les composés de formule (1) X - CF₂ - COOH, où X représente un atome d'halogène, et les composés de formule (2) R - G - CF₂-COOH, où R - G représente un groupe nitrile ou bien G représente >C = 0, >S = 0 ou -(CF₂)ₙ- avec n supérieur ou égal à 1 et R est un résidu organique ou minéral indifférent.

14. Procédé selon l'une quelconque des revendications précédentes, **caractérisé en ce que** ledit acide fluorocarboxylique au moins partiellement salifié dudit réactif est choisi parmi les sels d'acides perfluorocarboxyliques, notamment trifluoroacétate, perfluoropropionate et perfluorobutyrate de métal alcalin.

15. Procédé selon l'une quelconque des revendications précédentes, **caractérisé en ce que** ledit acide fluorocarboxylique, au moins partiellement salifié, dudit réactif est complètement soluble dans le milieu réactif.

16. Procédé selon l'une quelconque des revendications précédentes, **caractérisé en ce que** ledit acide dudit réactif est salifié par un cation de métal alcalin choisi parmi le sodium, le potassium, le rubidium, le césium et le francium, ou par un ammonium quaternaire.

17. Procédé selon l'une quelconque des revendications précédentes, **caractérisé en ce que** le solvant dudit réactif est choisi parmi les amides N-disubstitués, y compris les urées tétrasubstituées et les lactames monosubstitués, et les éthers cycliques ou non, et le benzonitrile.

18. Procédé selon l'une quelconque des revendications précédentes, **caractérisé en ce que** ledit oxyde de soufre est du bioxyde de soufre.

19. Procédé selon l'une quelconque des revendications précédentes, **caractérisé en ce que** le mélange liquide de l'étape a) est en équilibre avec une phase gazeuse contenant du bioxyde de soufre.

20. Procédé selon l'une quelconque des revendications précédentes, **caractérisé en ce que** l'on sépare les produits réactionnels alors que le taux de transformation de l'acide fluorocarboxylique est de 40 à 80 %.

21. Procédé selon l'une quelconque des revendications précédentes, **caractérisé en ce qu'**il comporte une étape c) d'oxydation du sel d'acide sulfinique obtenu à l'étape b), par mise en présence du produit de l'étape b) avec un réactif d'oxydation.

22. Réactif **caractérisé :**
- **en ce qu'**il est susceptible de former des dérivés organiques oxysulfurés et fluorés par réaction avec un oxyde de soufre, notamment du bioxyde de soufre,
- **en ce qu'**il comprend :
à) un acide fluorocarboxylique de formule Ea - CF₂ - COOH, au moins partiellement salifié par un cation choisi parmi les cations de métaux alcalins de rang supérieur à celui du sodium et les ammoniums ou phosphoniums quaternaires, où Ea représente un atome ou un groupe électroattracteur
b) un solvant aprotique ; et
- **en ce que** la teneur en protons libérables portés par ses divers composants, y compris leurs impuretés, est au plus égale à la moitié de la concentration molaire initiale dudit acide fluorocarboxylique; pour autant que le réactif ne consiste pas en un mélange de trifluoroacétate de potassium et de 1,3,5-trinitrobenzène dans le DMF ou le DMSO.

23. Réactif selon la revendication 22, **caractérisé en ce que** ladite teneur en protons est au plus égale à 10% de la concentration molaire en ledit acide fluorocarboxylique.

24. Réactif selon la revendication 22, **caractérisé en ce que** sa teneur en eau est inférieure à 10 % de la concentration molaire en ledit acide fluorocarboxylique.

25. Réactif selon l'une quelconque des revendications 22 à 24, **caractérisé en ce que** sa teneur en éléments de transition présentant au moins deux états de valence stables est inférieure à 1000 ppm molaires, avantageusement 100 ppm molaires, de préférence 10 ppm molaires par rapport audit acide fluorocarboxylique.

26. Réactif selon l'une quelconque des revendications 22 à 25, **caractérisé en ce que** sa teneur en éléments de la colonne VIII de la classification périodique des éléments est inférieure à 100 ppm molaires, par rapport audit acide fluorocarboxylique.

27. Réactif selon l'une quelconque des revendications 22 à 26, **caractérisé en ce que** sa teneur, exprimée en équivalent, en fluorure ionique est au plus égale à la concentration molaire dudit acide fluorocarboxylique.

28. Réactif selon la revendication 27 **caractérisé en ce que** le contre-cation du fluorure est choisi parmi les cations de métaux alcalins de rang supérieur à celui du sodium, en particulier potassium ou césium, ou bien les ammoniums ou phosponiums quaternaires.

29. Réactif selon l'une quelconque des revendications 22 à 28, **caractérisé en ce que** l'indice donneur dudit solvant aprotique est compris entre 10 et 30.

30. Réactif selon l'une quelconque des revendications 22 à 29, **caractérisé en ce que** l'indice accepteur dudit solvant est inférieur à 20.

31. Réactif selon l'une quelconque des revendications 22 à 30, **caractérisé en ce que** le pKa correspondant à la première acidité dudit solvant est au moins égal à 20.

32. Réactif selon l'une quelconque des revendications 22 à 31, **caractérisé en ce qu'**il comprend un éther couronne séquestrant.

33. Réactif selon l'une quelconque des revendications 22 à 32, **caractérisé en ce que** ledit atome ou .groupement électroattracteur est choisi parmi les groupes électroattracteurs dont la constante de Hammett σₚ est au moins égale à 0,1.

34. Réactif selon l'une quelconque des revendications 22 à 33, **caractérisé en ce que** ledit acide est choisi parmi les composés de formule (1) X - CF₂ - COOH, où X représente un atome d'halogène, et les composés de formule (2) R - G - CF₂-COOH, où R - G représente un groupe nitrile ou bien G représente >C = 0, >S = 0 ou -(CF₂)ₙ- avec n supérieur ou égal à 1 et R est un résidu organique ou minéral indifférent.

35. Réactif selon l'une quelconque des revendications 22 à 34, **caractérisé en ce que** ledit acide fluorocarboxylique au moins partiellement salifié est choisi parmi les sels d'acides perfluorocarboxyliques, notamment trifluoroacétate, perfluoropropionate et perfluorobutyrate de métal alcalin.

36. Réactif selon l'une quelconque des revendications 22 à 35, **caractérisé en ce que** ledit acide fluorocarboxylique, au moins partiellement salifié, est complètement soluble dans le milieu réactif.

37. Réactif selon l'une quelconque des revendications 22 à 36, **caractérisé en ce que** ledit acide est salifié par un cation de métal alcalin choisi parmi le sodium, le potassium, le rubidium, le césium et le francium, ou par un ammonium quaternaire.

38. Réactif selon l'une quelconque des revendications 22 à 37, **caractérisé en ce que** le solvant est choisi parmi les amides N-disubstitués, y compris les urées tétrasubstituées et les lactames monosubstitués, et les éthers cycliques ou non, et le benzonitrile.

39. Utilisation d'un réactif comprenant :
a) un acide fluorocarboxylique de formule
Ea - CF₂ - COOH, où Ea représente un atome ou un groupe électroattracteur, au moins partiellement salifié par un cation organique ou minéral, et
b) un solvant aprotique ;
dans lequel la teneur en protons libérables portés par les divers composants, y compris leurs impuretés, est au plus égale à la moitié de la concentration molaire initiale dudit acide fluorocarboxylique, pour la synthèse de dérivés organiques oxysulfurés et fluorés a partir d'un oxyde de soufre, notamment le bioxyde de soufre.

40. Milieu réactionnel comprenant :
- un oxyde de soufre, notamment du bioxyde de soufre, et
- un réactif pour la synthèse d'un dérivé fluoré dudit oxyde de soufre, ledit réactif comprenant :
a) un acide fluorocarboxylique de formule Ea - CF₂ - COOH, où Ea représente un atome ou un groupe électroattracteur, au moins partiellement salifié par un cation organique ou minéral, et
b) un solvant aprotique ;
et ayant une teneur en protons libérables portés par ses divers composants, y compris leurs impuretés, au plus égale à la moitié de la concentration molaire initiale dudit acide fluorocarboxylique.

## Claims

1. Process for the synthesis of a salt of an organic compound, which is both fluorinated and sulphoxylated, **characterised in that** it comprises the following steps:
a) bringing a reagent composed of:
a) a fluorocarboxylic acid with the formula
Ea - CF₂ - COOH, where Ea represents an electrically attracting atom or group at least partially salified by an organic or mineral cation, and
b) an aprotic solvent;
**in that** the content of releasable protons carried by their various components, including their impurities, is at most equal to half the initial molar concentration of said fluorocarboxylic acid,
together with a sulphur oxide; and
b) heating the resulting mixture at a temperature in the range of between 100 and 200°C for a period in the range of between a 1/2 hour to 20 hours.

2. Process according to Claim 1, **characterised in that** said proton content of the reagent is at most equal to 10% of the molar concentration in said fluorocarboxylic acid.

3. Process according to Claim 1, **characterised in that** the water content of said reagent is less than 10% of the molar concentration in said fluorocarboxylic acid.

4. Process according to any one of Claims 1 to 3, **characterised in that** the molar content of transition elements with at least two stable valency states of said reagent is less than 1000 ppm, advantageously 100 ppm, preferably 10 ppm in relation to said fluorocarboxylic acid.

5. Process according to any one of the preceding claims, **characterised in that** the molar content of elements from column VIII of the periodic classification of elements of said reagent is less than 100 ppm in relation to said fluorocarboxylic acid.

6. Process according to any one of the preceding claims, **characterised in that** the content, expressed as equivalent, of ionic fluoride of said reagent is at most equal to the molar concentration of said fluorocarboxylic acid.

7. Process according to Claim 6, **characterised in that** the counter-cation of the fluoride of said reagent is selected from among the cations of alkali metals ranked higher than sodium, in particular potassium or caesium, or "onium" type ions.

8. Process according to any one of the preceding claims, **characterised in that** the donor index of the aprotic solvent of said reagent is in the range of between 10 and 30.

9. Process according to any one of the preceding claims, **characterised in that** the acceptor index of the aprotic solvent of said reagent is less than 20.

10. Process according to any one of the preceding claims, **characterised in that** the pKa corresponding to the first acidity of the aprotic solvent of said reagent is at least equal to 20.

11. Process according to any one of the preceding claims, **characterised in that** said reagent contains a sequestrant crown ether.

12. Process according to any one of the preceding claims, **characterised in that** the electrically attracting atom or group of said reagent is selected from among the electrically attracting groups, wherein the Hammett constant σₚ is at least equal to 0.1.

13. Process according to any one of the preceding claims, **characterised in that** said acid of said reagent is selected from among compounds with the formula (1) X - CF₂ - COOH, where X represents a halogen atom, and compounds of formula (2) R - G - COOH, wherein R - G represents a nitrile group or G represents >C = 0, >S = 0 or -(CF₂)ₙ- where n is greater than or equal to 1 and R is an inert organic or mineral residue.

14. Process according to any one of the preceding claims, **characterised in that** said at least partially salified fluorocarboxylic acid of said reagent is selected from among the salts of perfluorocarboxylic acids, in particular alkali metal trifluoroacetate, perfluoropropionate and perfluorobutyrate.

15. Process according to any one of the preceding claims, **characterised in that** said partially salified fluorocarboxylic acid of said reagent is completely soluble in the reagent medium.

16. Process according to any one of the preceding claims, **characterised in that** said acid of said reagent is salified by a cation of an alkali metal selected from among sodium, potassium, rubidium, caesium and francium, or by a quaternary ammonium.

17. Process according to any one of the preceding claims, **characterised in that** the solvent of said reagent is selected from among the N-disubstituted amides including tetra-substituted ureas and monosubstituted lactams and cyclic or non-cyclic ethers and benzonitrile.

18. Process according to any one of the preceding claims, **characterised in that** said sulphur oxide is sulphur dioxide.

19. Process according to any one of the preceding claims, **characterised in that** the liquid mixture of step a) is in equilibrium with a gaseous phase containing sulphur dioxide.

20. Process according to any one of the preceding claims, **characterised in that** the reaction products are separated when the transformation rate of the fluorocarboxylic acid is 40 to 80%.

21. Process according to any one of the preceding claims, **characterised in that** it comprises a step c) of oxidising the sulphinic acid salt obtained in step b) by bringing the product of step b) together with an oxidation reagent.

22. Reagent **characterised**
• **in that** it is capable of forming sulphoxylated and fluorinated organic derivatives through reaction with a sulphur oxide, in particular sulphur dioxide;
• **in that** it is composed of:
a) a fluorocarboxylic acid with the formula
Ea - CF₂ - COOH at least partially salified by a cation selected from among the cations of alkali metals ranked higher than sodium and quaternary ammoniums or phosphoniums, where Ea represents an electrically attracting atom or group, and
b) an aprotic solvent; and
• **in that** the content of releasable protons carried by their various components, including their impurities, is at most equal to half the initial molar concentration of said fluorocarboxylic acid; while the reagent does not consist of a mixture of potassium trifluoroacetate and 1,3,5-trinitrobenzene in DMF or DMSO.

23. Reagent according to Claim 22, **characterised in that** said proton content is at most equal to 10% of the molar concentration in said fluorocarboxylic acid.

24. Reagent according to Claim 22, **characterised in that** its water content is less than 10% of the molar concentration in said fluorocarboxylic acid.

25. Reagent according to any one of Claims 22 to 24, **characterised in that** its molar content of transition elements with at least two stable valency states is less than 1000 ppm, advantageously 100 ppm, preferably 10 ppm in relation to said fluorocarboxylic acid.

26. Reagent according to any one of Claims 22 to 25, **characterised in that** its molar content of elements from column VIII of the periodic classification of elements is less than 100 ppm in relation to said fluorocarboxylic acid.

27. Reagent according to any one of Claims 22 to 26, **characterised in that** its content, expressed as equivalent, of ionic fluoride is at most equal to the molar concentration of said fluorocarboxylic acid.

28. Reagent according to Claim 27, **characterised in that** the counter-cation of the fluoride is selected from among the cations of alkali metals ranked higher than sodium, in particular potassium or caesium, or quaternary ammoniums or phosphoniums.

29. Reagent according to any one of Claims 22 to 28, **characterised in that** the donor index of said aprotic solvent is in the range of between 10 and 30.

30. Process according to any one of Claims 22 to 29, **characterised in that** the acceptor index of said aprotic solvent is less than 20.

31. Reagent according to any one of Claims 22 to 30, **characterised in that** the pKa corresponding to the first acidity of said aprotic solvent is at least equal to 20.

32. Reagent according to any one of Claims 22 to 31, **characterised in that** it contains a sequestrant crown ether.

33. Reagent according to any one of Claims 22 to 32, **characterised in that** said electrically attracting atom or group is selected from among the electrically attracting groups, wherein the Hammett constant σₚ is at least equal to 0.1.

34. Reagent according to any one of Claims 22 to 33, **characterised in that** said acid is selected from among compounds with the formula (1) X - CF₂ - COOH, where X represents a halogen atom, and compounds of formula (2) R - G - COOH, wherein R - G represents a nitrile group or G represents >C = 0, >S = 0 or -(CF₂)ₙ- where n is greater than or equal to 1 and R is an inert organic or mineral residue.

35. Reagent according to any one of Claims 22 to 34, **characterised in that** said at least partially salified fluorocarboxylic acid is selected from among the salts of perfluorocarboxylic acids, in particular alkali metal trifluoroacetate, perfluoropropionate and perfluorobutyrate.

36. Reagent according to any one of Claims 22 to 35, **characterised in that** said partially salified fluorocarboxylic acid is completely soluble in the reagent medium.

37. Reagent according to any one of Claims 22 to 36, **characterised in that** said acid is salified by a cation of an alkali metal selected from among sodium, potassium, rubidium, caesium and francium, or by a quaternary ammonium.

38. Reagent according to any one of Claims 22 to 37, **characterised in that** the solvent is selected from among the N-disubstituted amides including tetra-substituted ureas and monosubstituted lactams and cyclic or non-cyclic ethers and benzonitrile.

39. Use of a reagent composed of:
a) a fluorocarboxylic acid with the formula
Ea - CF₂ - COOH, where Ea represents an electrically attracting atom or group at least partially salified by an organic or mineral cation, and
b) an aprotic solvent;
in which the content of releasable protons carried by their various components, including their impurities, is at most equal to half the initial molar concentration of said fluorocarboxylic acid, for the synthesis of sulphoxylated and fluorinated organic derivatives from a sulphur oxide, in particular sulphur dioxide.

40. Reagent medium composed of:
• a sulphur oxide, in particular sulphur dioxide and
• a reagent for the synthesis of a fluorinated derivative of said sulphur oxide, said reagent being composed of:
a) a fluorocarboxylic acid with the formula
Ea - CF₂ - COOH, where Ea represents an electrically attracting atom or group at least partially salified by an organic or mineral cation, and
b) an aprotic solvent;
and having a content of releasable protons carried by their various components, including their impurities, at most equal to half the initial molar concentration of said fluorocarboxylic acid.

## Patentansprüche

1. Verfahren zur Synthese eines Salzes einer zugleich fluorierten und oxisulfurierten organischen Verbindung, **dadurch gekennzeichnet, dass** es die Schritte umfasst:
a) Zusammenbringen eines Reagens, umfassend:
a) eine mindestens teilweise durch ein organisches oder mineralisches Kation in ein Salz überführte Fluorcarbonsäure der Formel Ea-CF₂₋COOH, in der Ea ein elektrophiles Atom oder eine elektrophile Gruppe darstellt, und
b) ein aprotisches Lösungsmittel;
und dass der Gehalt an freisetzbaren Protonen, die von ihren verschiedenen Bestandteilen einschließlich ihren Verunreinigungen getragen werden, höchstens gleich der Hälfte der molaren Anfangskonzentration der Fluorcarbonsäure ist,
mit einem Schwefeloxid; und
b) Erhitzen der gebildeten Mischung auf eine Temperatur zwischen 100 und 200 °C während einer Zeit von 1/2 Stunde bis 20 Stunden.

2. Verfahren nach Anspruch 1, **dadurch gekennzeichnet, dass** der Gehalt des Reagens an Protonen höchstens gleich 10 % der molaren Konzentration an dieser Fluorcarbonsäure ist.

3. Verfahren nach Anspruch 1, **dadurch gekennzeichnet, dass** der Wassergehalt des Reagens kleiner als 10 % der molaren Konzentration an Fluorcarbonsäure ist.

4. Verfahren nach einem der Ansprüche 1 bis 3, **dadurch gekennzeichnet, dass** der Gehalt des Reagens an Übergangselementen mit mindestens zwei stabilen Wertigkeitszuständen kleiner als 1000 molare ppm, vorteilhafterweise 100 molare ppm und vorzugsweise 10 molare ppm, bezogen auf die Fluorcarbonsäure, ist.

5. Verfahren nach einem der vorhergehenden Ansprüche, **dadurch gekennzeichnet, dass** der Gehalt des Reagens an Elementen der Spalte VIII des periodischen Systems der Elemente kleiner als 100 molare ppm, bezogen auf die Fluorcarbonsäure, ist.

6. Verfahren nach einem der vorhergehenden Ansprüche, **dadurch gekennzeichnet, dass** der Gehalt des Reagens an ionischem Fluorid, ausgedrückt in Äquivalent, höchstens gleich der molaren Konzentration der Fluorcarbonsäure ist.

7. Verfahren nach Anspruch 6, **dadurch gekennzeichnet, dass** das Gegenkation des Fluorids des Regens aus den Kationen von Alkalimetallen höherer Ordnung als Natrium, insbesondere Kalium oder Caesium, oder den Ionen vom Typ "Onium", ausgewählt ist.

8. Verfahren nach einem der vorhergehenden Ansprüche, **dadurch gekennzeichnet, dass** die Donatorzahl des aprotischen Lösungsmittels des Reagens zwischen 10 und 30 beträgt.

9. Verfahren nach einem der vorhergehenden Ansprüche, **dadurch gekennzeichnet, dass** die Akzeptorzahl des aprotischen Lösungsmittels des Reagens kleiner als 20 ist.

10. Verfahren nach einem der vorhergehenden Ansprüche, **dadurch gekennzeichnet, dass** der pKa-Wert, der der ersten Azidität des aprotischen Lösungsmittels des Reagens entspricht, mindestens gleich 20 ist.

11. Verfahren nach einem der vorhergehenden Ansprüche, **dadurch gekennzeichnet, dass** das Reagens einen sequestrierenden Ringether umfasst.

12. Verfahren nach einem der vorhergehenden Ansprüche, **dadurch gekennzeichnet, dass** das elektrophile Atom oder die elektrophile Gruppe des Reagens ausgewählt ist aus den elektrophilen Gruppen, deren Hammettkonstante σₚ mindestens gleich 0,1 ist.

13. Verfahren nach einem der vorhergehenden Ansprüche, **dadurch gekennzeichnet, dass** die Säure des Reagens ausgewählt ist aus den Verbindungen der Formel (1) X-CF₂-COOH, in der X ein Halogenatom darstellt, und den Verbindungen der Formel (2) R-G-CF₂-COOH, in der R-G eine Nitrilgruppe darstellt oder G >C=0, >S=0 oder -(CF₂)ₙ- darstellt, wobei n größer als oder gleich 1 ist und R ein indifferenter organischer oder mineralischer Rest ist.

14. Verfahren nach einem der vorhergehenden Ansprüche, **dadurch gekennzeichnet, dass** die mindestens teilweise in ein Salz überführte Fluorcarbonsäure des Reagens ausgewählt ist aus den Perfluorcarbonsäuresalzen, insbesondere Trifluoracetat, Perfluorpropionat und Perfluorbutyrat eines Alkalimetalls.

15. Verfahren nach einem der vorhergehenden Ansprüche, **dadurch gekennzeichnet, dass** die mindestens teilweise in ein Salz überführte Fluorcarbonsäure des Reagens im Reaktionsmedium vollständig lösbar ist.

16. Verfahren nach einem der vorhergehenden Ansprüche, **dadurch gekennzeichnet, dass** die Säure des Reagens durch ein Alkalimetall-Kation, das aus Natrium, Kalium, Rubidium, Caesium und Francium ausgewählt ist, oder durch ein quaternäres Ammonium in ein Salz überführt ist.

17. Verfahren nach einem der vorhergehenden Ansprüche, **dadurch gekennzeichnet, dass** das Lösungsmittel des Reagens aus den N-disubstituierten Amiden, einschließlich den tetrasubstituierten Harnstoffen und den monosubstituierten Lactamen, und den zyklischen oder nicht zyklischen Ethern und Benzonitril ausgewählt ist.

18. Verfahren nach einem der vorhergehenden Ansprüche, **dadurch gekennzeichnet, dass** das Schwefeloxid Schwefeldioxid ist.

19. Verfahren nach einem der vorhergehenden Ansprüche, **dadurch gekennzeichnet, dass** die flüssige Mischung des Schritts a) mit einer Schwefeldioxid enthaltenden Gasphase im Gleichgewicht ist.

20. Verfahren nach einem der vorhergehenden Ansprüche, **dadurch gekennzeichnet, dass** man die Reaktionsprodukte trennt, während der Umwandlungsgrad der Fluorcarbonsäure 40 bis 80 % beträgt.

21. Verfahren nach einem der vorhergehenden Ansprüche, **dadurch gekennzeichnet, dass** es einen Schritt c) der Oxidation des in Schritt b) erhaltenen Sulfinsäuresalzes durch Zusammenbringen des Produkts des Schritts b) mit einem Oxidationsreagens umfasst.

22. Reagens, **dadurch gekennzeichnet,**
- **dass** es oxisulfurierte und fluorierte organische Derivate durch Reaktion mit einem Schwefeloxid, insbesondere Schwefeldioxid, bilden kann,
- **dass** es umfasst:
a) eine Fluorcarbonsäure der Formel Ea-CF₂-COOH, die mindestens teilweise durch ein Kation, das aus den Kationen von Alkalimetallen höherer Ordnung als Natrium und den quaternären Ammoniums und Phosphoniums ausgewählt ist, in ein Salz überführt ist, wobei Ea ein elektrophiles Atom oder eine elektrophile Gruppe darstellt;
b) ein aprotisches Lösungsmittel; und
- **dass** der Gehalt an freisetzbaren Protonen, die von seinen verschiedenen Bestandteilen einschließlich ihren Verunreinigungen getragen werden, höchstens gleich der Hälfte der molaren Anfangskonzentration der Fluorcarbonsäure ist, sofern das Reagens nicht aus einer Mischung aus Kaliumtrifluoracetat und 1,3,5-Trinitrobenzol in DMF oder DMSO besteht.

23. Reagens nach Anspruch 22, **dadurch gekennzeichnet, dass** der Gehalt an Protonen höchstens gleich 10 % der molaren Konzentration an Fluorcarbonsäure ist.

24. Reagens nach Anspruch 22, **dadurch gekennzeichnet, dass** sein Gehalt an Wasser niedriger als 10 % der molaren Konzentration an Fluorcarbonsäure ist.

25. Reagens nach einem der Ansprüche 22 bis 24, **dadurch gekennzeichnet, dass** sein Gehalt an Übergangselementen mit mindestens zwei stabilen Wertigkeitszuständen kleiner als 1000 molare ppm, vorzugsweise 10 molare ppm, bezogen auf die Fluorcarbonsäure, ist.

26. Reagens nach einem der Ansprüche 22 bis 25, **dadurch gekennzeichnet, dass** sein Gehalt an Elementen der Spalte VIII des periodischen Systems der Elemente kleiner als 100 molare ppm, bezogen auf die Fluorcarbonsäure, ist.

27. Reagens nach einem der Ansprüche 22 bis 26, **dadurch gekennzeichnet, dass** sein in Äquivalent ausgedrückter Gehalt an ionischem Fluorid höchstens gleich der molaren Konzentration der Fluorcarbonsäure ist.

28. Reagens nach Anspruch 27, **dadurch gekennzeichnet, dass** das Gegenkation des Fluorids ausgewählt ist aus den Kationen von Alkalimetallen höherer Ordnung als Natrium, insbesondere Kalium oder Caesium, oder den quaternären Ammoniums oder Phosphoniums.

29. Reagens nach einem der Ansprüche 22 bis 28, **dadurch gekennzeichnet, dass** die Donatorzahl des aprotischen Lösungsmittels 10 bis 30 beträgt.

30. Reagens nach einem der Ansprüche 22 bis 29, **dadurch gekennzeichnet, dass** die Akzeptorzahl des Lösungsmittels kleiner als 20 ist.

31. Reagens nach einem der Ansprüche 22 bis 30, **dadurch gekennzeichnet, dass** der der ersten Azidität des Lösungsmittels entsprechende pKa-Wert mindestens gleich 20 ist.

32. Reagens nach einem der Ansprüche 22 bis 31, **dadurch gekennzeichnet, dass** es einen sequestrierenden Ringether umfasst.

33. Reagens nach einem der Ansprüche 22 bis 32, **dadurch gekennzeichnet, dass** das elektrophile Atom oder die elektrophile Gruppe ausgewählt ist aus den elektrophilen Gruppen, deren Hammettkonstante σₚ mindestens gleich 0,1 ist.

34. Reagens nach einem der Ansprüche 22 bis 33, **dadurch gekennzeichnet, dass** die Säure ausgewählt ist aus den Verbindungen der Formel (1) X-CF₂-COOH, in der X ein Halogenatom darstellt, und den Verbindungen der Formel (2) R-G-CF₂-COOH, in der R-G eine Nitrilgruppe darstellt oder G >C=0, >S=0 oder - (CF₂)ₙ- darstellt, wobei n größer als oder gleich 1 und R ein indifferenter organischer oder mineralischer Rest ist.

35. Reagens nach einem der Ansprüche 22 bis 34, **dadurch gekennzeichnet, dass** die mindestens teilweise in ein Salz überführte Fluorcarbonsäure ausgewählt ist aus den Salzen von Perfluorcarbonsäuren, insbesondere Trifluoracetat, Perfluorpropionat und Perfluorbutyrat eines Alkalimetalls.

36. Reagens nach einem der Ansprüche 32 bis 35, **dadurch gekennzeichnet, dass** die mindestens teilweise in ein Salz überführte Fluorcarbonsäure im Reaktionsmedium vollständig lösbar ist.

37. Reagens nach einem der Ansprüche 22 bis 36, **dadurch gekennzeichnet, dass** die Säure durch ein Alkalimetall-Kation, das aus Natrium, Kalium, Rubidium, Caesium und Francium ausgewählt ist, oder durch ein quaternäres Ammonium in ein Salz überführt ist.

38. Reagens nach einem der Ansprüche 22 bis 37, **dadurch gekennzeichnet, dass** das Lösungsmittel des Reagens aus den N-disubstituierten Amiden, einschließlich den tetrasubstituierten Harnstoffen und den monosubstituierten Lactamen, und den zyklischen oder nicht zyklischen Ethern und Benzonitril ausgewählt ist.

39. Verwendung eines Reagens, umfassend
a) eine mindestens teilweise durch ein organisches oder mineralisches Kation in ein Salz überführte Fluorcarbonsäure der Formel Ea-CF₂₋COOH, in der Ea ein elektrophiles Atom oder eine elektrophile Gruppe darstellt;
b) ein aprotisches Lösungsmittel;
- bei dem der Gehalt an freisetzbaren Protonen, die von seinen verschiedenen Bestandteilen einschließlich ihren Verunreinigungen getragen werden, höchstens gleich der Hälfte der molaren Anfangskonzentration der Fluorcarbonsäure ist, für die Synthese von oxisulfurierten und fluorierten organischen Derivaten aus einem Schwefeloxid, insbesondere Schwefelbioxid.

40. Reaktionsmedium, das umfasst
- ein Schwefeloxid, insbesondere Schwefeldioxid, und
- ein Reagens für die Synthese eines fluorierten Derivats dieses Schwefeloxids, wobei dieses Reagens umfasst:
a) eine mindestens teilweise durch ein organisches oder mineralisches Kation in ein Salz überführte Fluorcarbonsäure der Formel Ea-CF₂₋COOH, in der Ea ein elektrophiles Atom oder eine elektrophile Gruppe darstellt, und
b) ein aprotisches Lösungsmittel;
und das einen Gehalt an freisetzbaren Protonen, die von seinen verschiedenen Bestandteilen einschließlich ihren Verunreinigungen getragen werden, höchstens gleich der Hälfte der molaren Anfangskonzentration der Fluorcarbonsäure ist.
